# DEMANDE DE BREVET EUROPEEN

(11) **EP 2 848 277 A1**
(43) Date de publication de la demande: **18.03.2015**
(21) Numéro de dépôt: 14306283.4
(22) Date de dépôt: 18.08.2014
(51) Int. Cl.: A61M 16/16

(54) **Cuve à liquide pour humidificateur de gaz médical**

(30) Priorité: 12.09.2013 FR 1358765
(71) Demandeur: Air Liquide Medical Systems, 92160 Antony (FR)
(72) Inventeur: Davoine, Romain, 75014 PARIS (FR)
(74) Mandataire: Pittis, Olivier

(57) **Abrégé**

L'invention porte sur une cuve (1) à liquide pour humidificateur de gaz comprenant un fond (2) et une paroi périphérique (3) définissant un volume intérieur (4) apte à contenir un liquide, ledit fond (2) comportant une plaque diffuseur thermique (5) en contact avec le volume intérieur (4), caractérisée en ce que la plaque diffuseur thermique (5) est fixée au fond (2) de la cuve (1) par l'intermédiaire d'un élément élastique (6) en un matériau déformable. L'invention concerne également un humidificateur (11) de gaz comprenant une cuve (1) à liquide, en particulier à eau, selon l'invention, de préférence une cuve (1) amovible, ainsi qu'une installation de fourniture de gaz respiratoire humidifié à un patient (P) comprenant un appareil d'assistance respiratoire (10) apte à délivrer un gaz respiratoire, relié fluidiquement (14) à une entrée de gaz (21) d'un tel humidificateur de gaz (11).

## Description

La présente invention concerne une cuve à liquide amovible pour humidificateur de gaz apte à contenir un liquide, en particulier de l'eau, incluant une plaque diffuseur thermique fixée par un élément élastique, et un humidificateur de gaz incorporant une telle cuve.

Les systèmes d'humidification de gaz actuels, encore appelés humidificateurs de gaz, comprennent un boitier ou capotage contenant une source chauffante et une cuve d'eau fixe ou amovible incorporant une plaque diffuseur thermique.

La source chauffante est habituellement intégrée dans le fond du boitier de l'humidificateur. Elle comprend une plaque chauffante équipée d'un élément chauffant (souple, filaire...) apte à s'échauffer en émettant des calories lorsqu'il est soumis à un courant électrique, et d'éléments électroniques de contrôle/pilotage et de sécurité, tels que sonde thermique, thermistance... servant à piloter la source chauffante notamment.

Par ailleurs, la cuve ou bac-réservoir à liquide est formée d'une ou plusieurs pièces et est destinée à contenir un volume de liquide, typiquement d'eau.

En fonctionnement, la plaque chauffante agencée dans le boitier de l'humidificateur émet des calories qui sont transmises à la plaque diffuseur thermique qui est en contact mécanique avec ladite plaque chauffante de la cuve. Ces calories sont transmises ensuite à l'eau qui est elle-même en contact avec la plaque diffuseur thermique. Ceci permet une humidification du gaz qui se mélange à la vapeur d'eau générée par chauffage de l'eau.

L'entrée de l'humidificateur est reliée à un générateur de flux, à savoir un ventilateur d'assistance respiratoire par exemple, qui délivre du gaz respiratoire à humidifier, alors que la sortie de l'humidificateur alimente, au travers d'un circuit patient, le patient en gaz respiratoire humidifié. Typiquement, le gaz humidifié dans l'humidificateur est de l'air ou de l'air enrichi en oxygène.

Le document WO-A-10140903 décrit un humidificateur de ce type comprenant une cuve d'eau amovible intégrant une plaque diffuseur thermique et un boitier avec plaque chauffante au sein duquel vient se positionner la cuve de manière à ce que la plaque chauffante soit au contact de la plaque diffuseur thermique. Dans ce cas, la plaque chauffante est maintenue dans le boitier de l'humidificateur par un élément élastique agencé, d'une part, à la périphérie de la plaque chauffante et, d'autre part, entre deux parois du boitier qui prennent cet élément élastique en sandwich.

Cette solution n'est pas idéale car elle est d'architecture compliquée puisqu'elle oblige à prévoir des aménagements spécifiques au sein du boitier de l'humidificateur lui-même, notamment de prévoir des parois pour prendre spécifiquement l'élément élastique en sandwich et le maintenir fermement en place.

De plus, l'élément élastique est dans la partie de l'appareil qui n'est pas interchangeable. Donc, en cas d'usure ou de vieillissement prématuré, c'est l'ensemble du boitier et/ou de son système de chauffe qui doit être mis en maintenance et/ou remplacé. Ceci engendre un surcoût non négligeable du fait du nombre important d'éléments à remplacer.

Le problème à résoudre est dès lors de proposer un humidificateur de conception améliorée ne présentant pas tout ou partie des problèmes susmentionnés.

La solution de l'invention est alors une cuve à liquide pour humidificateur de gaz comprenant un fond et une paroi périphérique définissant un volume intérieur apte à contenir un liquide, en particulier de l'eau, ledit fond comportant une plaque diffuseur thermique en contact avec le volume intérieur, caractérisée en ce que la plaque diffuseur thermique est fixée au fond de la cuve par l'intermédiaire d'un élément élastique en un matériau déformable.

Selon le cas, la cuve à liquide de l'invention peut comprendre l'une ou plusieurs des caractéristiques techniques suivantes :
- la plaque diffuseur thermique comprend un rebord périphérique externe et le fond de la cuve comprend une découpe comprenant un rebord interne, ledit élément élastique étant agencé le long dudit rebord périphérique de la plaque diffuseur thermique et le long dudit rebord interne du fond.
- le fond de la cuve comprend une découpe centrale.
- le fond de la cuve comprend une découpe de forme circulaire, oblongue ou rectangulaire.
- l'élément élastique a une forme générale en couronne.
- la cuve est formée d'un matériau plastique rigide, de préférence thermoplastique.
- la plaque diffuseur thermique est en un matériau métallique, de préférence en aluminium ou un alliage d'aluminium, ou en acier inoxydable, ou en plastique conducteur, c'est-à-dire un matériau thermoplastique ayant des propriétés de conduction thermique équivalentes ou similaires au métal.
- l'élément élastique est formé d'un matériau déformable choisi parmi les matériaux thermoplastiques élastomères, le caoutchouc et les élastomères à base de silicone.
- l'élément élastique est fixé au rebord périphérique de la plaque diffuseur thermique et au rebord interne du fond par surmoulage ou par collage.
- ledit élément élastique comprend une extrémité de fixation proximale et une extrémité de fixation distale, lesdites extrémités de fixation proximale et distale venant prendre « en sandwich » le rebord interne du fond et le rebord périphérique de la plaque diffuseur thermique, respectivement.
- alternativement, l'élément élastique est fixé par adhésion, par exemple par collage, au rebord interne du fond et au rebord périphérique de la plaque diffuseur thermique, respectivement, de préférence au moins à la face supérieure du rebord interne du fond et/ou au moins à la face supérieure du rebord périphérique de la plaque diffuseur.

L'invention porte en outre sur un humidificateur de gaz comprenant une cuve à liquide selon l'invention, de préférence une cuve amovible.

Selon le cas, l'humidificateur de gaz de l'invention peut comprendre l'une ou plusieurs des caractéristiques techniques suivantes :
- la cuve contient de l'eau.
- il comporte en outre une plaque chauffante en contact avec la plaque diffuseur thermique de la cuve, c'est-à-dire lorsque la cuve est insérée dans l'humidificateur.
- la plaque chauffante est équipée d'un élément chauffant.
- la plaque chauffante équipée d'un élément chauffant est apte à et conçue pour émettre des calories, c'est-à-dire dégager de la chaleur, lorsqu'elle est alimentée en courant électrique.
- la plaque chauffante est reliée à une source de courant électrique, à savoir une source interne, telle une ou plusieurs batteries électriques, rechargeables ou non, ou une source externe, telle l'alimentation du secteur électrique.
- il comporte en outre une chambre d'humidification surmontant la cuve.
- il comprend une entrée de gaz et une sortie de gaz.
- l'entrée de gaz est reliée fluidiquement à la chambre d'humidification de manière à alimenter ladite chambre d'humidification en gaz à humidifier.
- la sortie de gaz est reliée fluidiquement à la chambre d'humidification de manière à extraire de ladite chambre d'humidification du gaz humidifié au sein de ladite chambre d'humidification.

Par ailleurs, l'invention porte aussi sur une installation de fourniture de gaz respiratoire humidifié à un patient comprenant un appareil d'assistance respiratoire, tel un ventilateur médical, apte à délivrer un gaz respiratoire, tel de l'air, ledit appareil étant relié fluidiquement à une entrée de gaz d'un humidificateur de gaz selon l'invention, de manière à l'alimenter en gaz délivré par ledit appareil d'assistance respiratoire.

L'installation comporte en outre au moins un conduit d'acheminement de gaz relié fluidiquement à une sortie de l'humidificateur de gaz de manière à récupérer et convoyer du gaz humidifié provenant, c'est-à-dire sortant, dudit humidificateur de gaz.

Le conduit d'acheminement de gaz alimente ensuite une interface patient, tel un masque respiratoire, des lunettes respiratoires ou une sonde d'intubation.

La présente invention va maintenant être mieux comprise grâce à la description suivante faite en référence aux Figures annexées parmi lesquelles :
- la Figure 1 représente un schéma d'un humidificateur de gaz avec une cuve de liquide selon la présente invention,
- la Figure 2 est une vue grossie de la zone de liaison entre cuve et plaque diffuseur thermique de la cuve de la Figure 1,
- la Figure 3 est une vue de côté d'un mode de réalisation d'un humidificateur selon le principe de la Figure 1, et
- la Figure 4 est un schéma d'une installation de fourniture de gaz respiratoire incorporant l'humidificateur de la Figure 3.

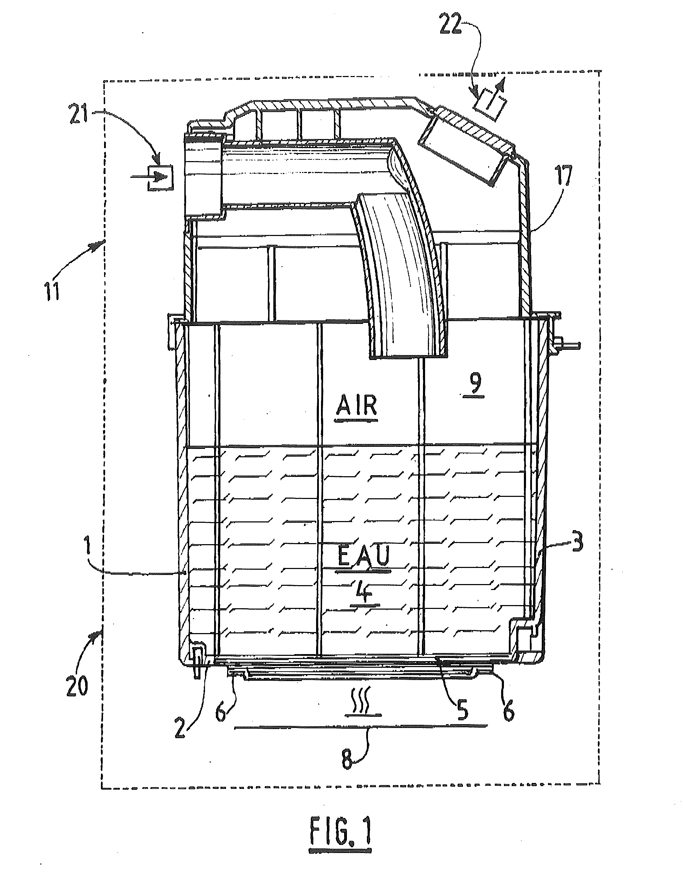

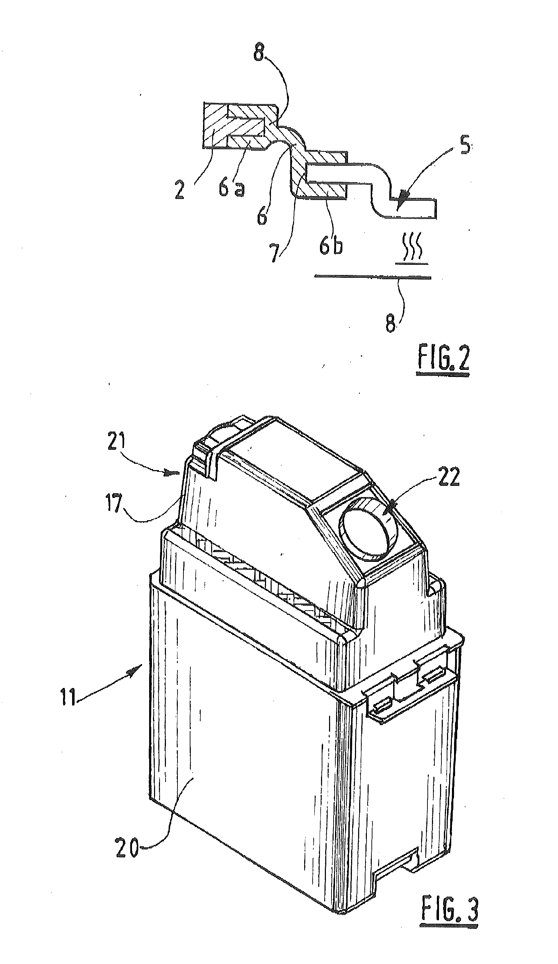

Les Figures 1 et 3 schématisent un humidificateur de gaz 11 avec une cuve à liquide, à savoir à eau, selon la présente invention et son principe de fonctionnement.

Plus précisément, l'humidificateur 11 de l'invention comprend un boitier 20 formant coque ou capotage externe, comprenant une entrée de gaz 21 et une sortie de gaz 22. Le gaz à humidifier, typiquement de l'air, entre dans l'humidificateur 11 par l'entrée 21 et en ressort, après humidification, par la sortie 22.

Entre lesdites entrée de gaz 21 et sortie de gaz 22, l'humidificateur 11 comprend une chambre d'humidification 9 surmontant une cuve 1 amovible contenant le liquide servant à l'humidification du gaz, à savoir typiquement de l'eau.

La cuve 1 est munie d'un fond 2 et une paroi périphérique 3 définissant ainsi un volume intérieur 4 au sein duquel est retenue l'eau servant à humidifier le gaz. La cuve 1 constitue donc une réserve d'eau d'humidification.

Le fond 2 de la cuve 1 comprend une ouverture ou découpe centrale dans ou au regard de laquelle est agencée une plaque diffuseur thermique 5 qui est elle-même en contact avec le volume intérieur, donc avec le liquide. Cette plaque diffuseur thermique 5 fait office d'échangeur thermique.

En fait, cette plaque diffuseur 5 est en contact mécanique, direct ou indirect, avec une plaque chauffante 8 agencée dans le boitier 20 de l'humidificateur 11, laquelle plaque chauffante 8 est équipée d'un élément chauffant souple ou rigide qui s'échauffe sous l'effet d'un courant électrique qui l'alimente. La source de courant électrique 15 peut être une source interne, telle une batterie rechargeable ou non, ou une source externe comme l'alimentation du secteur électrique.

L'élément chauffant souple ou rigide (e.g. rigide 8x) peut être installé directement sur la plaque diffuseur 5 et connecté à la source de courant électrique 15 via des contacts mobiles.

La plaque diffuseur thermique 5 et la plaque chauffante 8 sont thermiquement conductrices. Par exemple, la plaque diffuseur thermique 5 et/ou la plaque chauffante 8 est/sont en un métal ou alliage métallique, par exemple en acier inoxydable, en aluminium, en alliage d'aluminium ou en matériau thermoplastique conducteur.

Les calories émises par la plaque chauffante 8 sont alors transmises à la plaque diffuseur thermique 5 par échange thermique du fait du contact mécanique existant entre lesdites plaques 5, 8, puis passent à l'eau contenue dans la cuve 1 du fait du contact existant entre ladite plaque diffuseur thermique 5 et l'eau contenue dans le volume interne 4 de la cuve 1. En d'autres termes, la surface inférieure de plaque diffuseur thermique 5 est en contact avec la plaque chauffante 8, alors que sa surface supérieure est en contact avec l'eau à chauffer de sorte d'assurer, via ladite plaque diffuseur thermique 5, un passage d'énergie calorifique de la plaque chauffante 8 au liquide à chauffer.

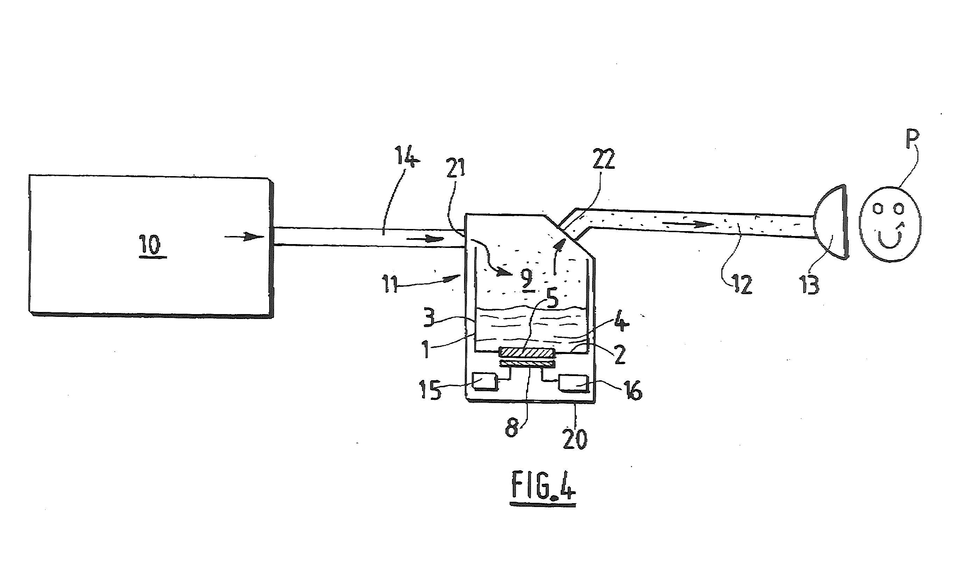

L'eau se vaporise, au moins partiellement, et la vapeur d'eau ainsi produite va se mélanger, au-dessus de la cuve 1 et dans la chambre d'humidification 9, au gaz, tel de l'air, qui alimente l'humidificateur 11. Il en résulte un gaz humidifié qui peut alors être envoyé à un patient P, comme illustré en Figure 4.

Selon la présente invention, la plaque diffuseur thermique 5 est fixée au fond 2 de la cuve 1 par l'intermédiaire d'un élément élastique 6 en un matériau souple et déformable.

Dit autrement, la liaison mécanique entre la plaque diffuseur thermique 5 et le fond 2 de la cuve est assurée par un élément élastique 6, c'est-à-dire un élément formé d'un matériau souple et déformable, qui assure dès lors également une fonction d'étanchéité fluidique entre plaque diffuseur thermique 5 et fond 2 de cuve, et permet en outre, du fait de sa souplesse et de sa déformabilité, de conférer une latitude de positionnement de la plaque diffuseur thermique 5 par rapport à la plaque chauffante 8 et donc permettre un contact mécanique efficace, voire optimum, entre elles, c'est-à-dire sans espacement ou jeu entre les plaques 5, 8.

Les plaques 5, 8 et l'élément élastique 6 sont liés au montage de l'ensemble. L'élément élastique 6 possède une géométrie particulière facilitant sa déformation et donc son effet ressort lors des mouvements de montage. L'élément élastique 6 peut être fixé par procédé de surmoulage, collage ou soudage thermique ou par ultrasons.

En d'autres termes, l'humidificateur 11 comprend un ensemble de plusieurs éléments qui forment la partie dite « réservoir » de l'humidificateur 11 au sein de laquelle vient se positionner la cuve 1. On accède à l'intérieur de cette partie « réservoir » en soulevant un couvercle 17 pivotant ou amovible recouvrant le haut de l'humidificateur 11.

Plus précisément, la partie dite « réservoir » est formée notamment de pièces thermoplastiques rigides constituant tout ou partie de l'enveloppe de la partie « réservoir » de l'humidificateur. Cette enveloppe du réservoir définit quant à elle un compartiment interne formé de :
- une partie « haute » située du côté du couvercle 17 comprenant la chambre d'humidification 9 et
- une partie « basse », située du côté du fond du compartiment-réservoir, qui accueille la cuve 1 servant de réserve d'eau.

La chambre d'humidification 9 et la cuve 1 sont donc aménagées sur le trajet du gaz entre le ventilateur 10 et le patient P, comme visible sur la Figure 4, et doivent pouvoir être démontées facilement, par exemple via des clips démontables ou tout autre système de fixation adapté, pour autoriser leur nettoyage ou leur remplacement.

Le compartiment-réservoir comprend des géométries de guidage du réservoir 1, ainsi que des butées permettant l'écrasement de la plaque diffuseur thermique 5 en facilitant et améliorant ainsi sa mise en contact avec la plaque chauffante 8.

Il est également prévu des pièces plastiques souples, en thermoplastique élastomère, en caoutchouc ou en silicone, permettant l'étanchéité des différentes parties entre elles, notamment des parties basse et haute, ainsi que l'étanchéité de l'entrée et la sortie du trajet du gaz. Pour ce faire, on peut agencer des joints surmoulés dans les pièces thermoplastiques rigides susmentionnées.

La pièce métallique ayant une forme de plaque, appelée plaque diffuseur thermique 5, permet l'échange thermique entre la plaque chauffante intégrée 8 au boitier humidificateur et le volume d'eau contenu dans le volume interne 4 de la cuve 1.

Comme déjà évoqué, l'élément élastique 6 est formé d'une pièce thermoplastique souple, encore appelée membrane, liant la plaque diffuseur thermique 5 et la partie basse ou fond 2 de la cuve 1.

L'élément élastique 6 est préférentiellement réalisé par un surmoulage entre le fond 2 de la cuve à liquide et la plaque diffuseur thermique 5.

Cet élément élastique 6 a donc pour rôle non seulement de réaliser une liaison mécanique entre la plaque diffuseur thermique 5 et le fond de la cuve 1 mais aussi d'assurer une étanchéité fluidique entre ces parties et de permettre un amortissement des plaques 5, 8, lors de leur mise en contact l'une avec l'autre, lors de l'insertion de la cuve 1 dans le fond de l'humidificateur 11.

Cet élément élastique 6 permet un montage optimum en production réalisé lors des phases de fabrication par injection thermoplastique rigide, puis injection thermoplastique souple, élastomère, avec la plaque diffuseur thermique 5, et non un montage mécanique lors de l'intégration du produit, c'est-à-dire réalisé par un opérateur, qui est, dans les appareils connus, générateur de défauts, tel que mauvaise étanchéité, défauts de géométrie des pièces...

La cuve 1 selon l'invention est réalisée en matériaux thermiquement résistants à la chauffe du volume d'eau et aux contraintes environnementales/utilisateur, tel que le nettoyage chimique, le nettoyage par passage au lave-vaisselle, la "biocompatibilité" des matériaux...

La Figure 2 est une vue grossie de la zone de liaison entre la cuve 1 et la plaque diffuseur thermique 5.

Comme on le voit, l'élément élastique 6 est fixé au rebord périphérique 7, c'est-à-dire au bord externe situé à la périphérie extérieure, de la plaque diffuseur thermique 5 et au rebord interne 8 du fond 2, c'est-à-dire au bord résulte d'une découpe ou ouverture pratiquée dans le fond 2 de la cuve 1.

En fait, l'élément élastique 6 comprend une extrémité de fixation proximale 6a et une extrémité de fixation distale 6b qui viennent prendre « en sandwich » le rebord interne 8 du fond 2 et le rebord périphérique 7 de la plaque diffuseur thermique 5, respectivement. L'élément élastique 6 vient donc se fixer au moins aux surfaces supérieures ou du dessus et inférieure ou du dessous du fond 2 et de la plaque 5.

Ceci peut être aisément obtenu par une technique de surmoulage.

La Figure 4 schématise une installation de fourniture de gaz respiratoire humidifié, à savoir de l'air humidifié, à un patient P comprenant un appareil d'assistance respiratoire 10, tel un ventilateur d'assistance médical, qui délivre le gaz respiratoire non-humidifié, par exemple au moyen d'une turbine ou analogue, lequel appareil est relié fluidiquement, via un conduit 14, à l'entrée de gaz 21 d'un humidificateur de gaz 11 incluant une cuve de liquide, c'est-à-dire d'eau, selon la présente invention, de manière à alimenter l'humidificateur 11 en gaz à humidifier au sein dudit humidificateur 11.

Un conduit d'acheminement de gaz 12 relié fluidiquement à la sortie 22 de l'humidificateur 11 de gaz permet de récupérer et de convoyer le gaz humidifié sortant de l'humidificateur 11, jusqu'à une interface patient 13.

Au sein de l'humidificateur 11, le gaz est humidifié par passage dans la chambre d'humidification 9 surmontant la cuve 1 à eau incluant la plaque diffuseur thermique 5 qui est chauffée par la plaque chauffante 8.

La plaque chauffante 8 est équipée d'un élément chauffant type 8x (souple, filaire...) qui s'échauffe en émettant des calories lorsqu'il est soumis à un courant électrique issu d'une source de courant 15, tel une ou des batteries électriques ou le secteur (110-230 V).

Le pilotage du chauffage est assuré par des moyens électroniques 16 de contrôle/pilotage et de sécurité, tels que sonde thermique, thermistance....

Comme déjà expliqué, pendant le fonctionnement, la plaque chauffante 8 émet des calories qui sont transmises à la plaque diffuseur thermique 5 qui est en contact mécanique avec ladite plaque chauffante de la cuve.

Ces calories sont ensuite transmises à l'eau située dans la cuve 1 et qui est elle-même en contact avec la plaque diffuseur thermique 5, de manière à vaporiser une partie de cette eau et à provoquer, notamment au sein de la chambre de mélange 9 surmontant la cuve 1, une humidification du gaz par mélange à la vapeur d'eau générée par le chauffage de l'eau.

Une partie du gaz s'humidifie aussi dans le ciel gazeux situé juste au-dessus de l'eau stockée dans la cuve 1.

Le gaz ainsi humidifié est ensuite convoyé vers le patient, de manière classique par un conduit de gaz et l'interface patient 13, tel un masque respiratoire ou analogue.

## Revendications

1. Cuve (1) à liquide pour humidificateur de gaz comprenant un fond (2) et une paroi périphérique (3) définissant un volume intérieur (4) apte à contenir un liquide, ledit fond (2) comportant une plaque diffuseur thermique (5) en contact avec le volume intérieur (4), **caractérisée en ce que** la plaque diffuseur thermique (5) est fixée au fond (2) de la cuve (1) par l'intermédiaire d'un élément élastique (6) en un matériau déformable.

2. Cuve selon la revendication précédente, **caractérisée en ce que** la plaque diffuseur thermique (5) comprend un rebord périphérique (7) externe et le fond (2) de la cuve (1) comprend une découpe comprenant un rebord interne (8), ledit élément élastique (6) étant agencé le long dudit rebord périphérique (7) de la plaque diffuseur thermique (5) et le long dudit rebord interne (8) du fond (2).

3. Cuve selon l'une des revendications précédentes, **caractérisée en ce que** l'élément élastique (6) a une forme générale en couronne.

4. Cuve selon l'une des revendications précédentes, **caractérisée en ce qu'**elle est formée d'un matériau plastique rigide, de préférence thermoplastique.

5. Cuve selon l'une des revendications précédentes, **caractérisée en ce que** la plaque diffuseur thermique (5) est en un matériau métallique choisi parmi l'acier inoxydable, l'aluminium et les alliages d'aluminium, ou en un plastique conducteur.

6. Cuve selon l'une des revendications précédentes, **caractérisée en ce que** l'élément élastique (6) est formé d'un matériau déformable choisi parmi les matériaux thermoplastiques élastomères, le caoutchouc et les élastomères à base de silicone.

7. Cuve selon l'une des revendications précédentes, **caractérisée en ce que** l'élément élastique (6) est fixé au rebord périphérique (7) de la plaque diffuseur thermique (5) et au rebord interne (8) du fond (2) par surmoulage ou par collage.

8. Cuve selon l'une des revendications précédentes, **caractérisée en ce que** l'élément élastique (6) comprend une extrémité de fixation proximale (6a) et une extrémité de fixation distale (6b), lesdites extrémités de fixation proximale (6a) et distale (6b) venant prendre en sandwich le rebord interne (8) du fond (2) et le rebord périphérique (7) de la plaque diffuseur thermique (5), respectivement.

9. Humidificateur (11) de gaz comprenant une cuve (1) à liquide, en particulier à eau, selon l'une des revendications précédentes, de préférence une cuve (1) amovible.

10. Humidificateur selon la revendication 9, **caractérisé en ce qu'**il comporte en outre une plaque chauffante (8) en contact avec la plaque diffuseur thermique (5) de la cuve (1) à liquide, lorsque la cuve (1) est insérée dans l'humidificateur.

11. Humidificateur selon l'une des revendications 9 ou 10, **caractérisé en ce qu'**il comporte en outre une chambre d'humidification (9) surmontant la cuve (1).

12. Installation de fourniture de gaz respiratoire humidifié à un patient (P) comprenant un appareil d'assistance respiratoire (10) apte à délivrer un gaz respiratoire, relié fluidiquement (14) à une entrée de gaz (21) d'un humidificateur de gaz (11) selon l'une des revendications 9 à 11, de manière à alimenter ledit humidificateur (11) en un gaz délivré par ledit appareil d'assistance respiratoire (10).

13. Installation de fourniture de gaz selon la revendication 12, **caractérisée en ce qu'**elle comporte en outre au moins un conduit d'acheminement de gaz (12) relié fluidiquement à une sortie (22) de l'humidificateur (11) de gaz de manière à récupérer et convoyer du gaz humidifié sortant de l'humidificateur (11) de gaz, de préférence ledit conduit (14) alimente une interface patient (13).
